Europäisches Patentamt

(19) European Patent Office

Office européen des brevets

(11) Publication number: **0 276 134**
A2

# (12) EUROPEAN PATENT APPLICATION

(21) Application number: 88300434.3

(22) Date of filing: 20.01.88

(51) Int. Cl.⁴: **G 01 N 33/68**
G 01 N 33/543, G 01 N 33/58
// G01N33/561, G01N33/544

(30) Priority: 20.01.87 US 4464   17.09.87 US 98324

(43) Date of publication of application:
27.07.88 Bulletin 88/30

(84) Designated Contracting States:
AT BE CH DE ES FR GB GR IT LI LU NL SE

(71) Applicant: INTERFERON SCIENCES, INC.
783 Jersey Avenue
New Brunswick New Jersey 08901 (US)

(72) Inventor: Lee, Nancy
6 Redwood Court
Lawrenceville New Jersey 08648 (US)

Yand, Jinshui
Oakleaf Village Apt. C-9
North Brunswick New Jersey 08902 (US)

(74) Representative: Bannerman, David Gardner et al
Withers & Rogers 4 Dyer's Buildings Holborn
London, EC1N 2JT (GB)

(54) Multicolor western blot.

(57) Multicolor Western and dot blots are provided, each color representing a different protein or group of proteins. In certain embodiments, the different colors are achieved by: (a) incubating the blot with a mixture of two unlabelled binding probes; (b) incubating the blot with a mixture of two enzyme-labelled marking probes; and (c) sequentially incubating the blot with substrates for the enzyme labels so as to produce two marker substances having different colors which bind to the blot at the locations of the marking probes. In certain embodiments, various of the incubations are combined so as to provide development protocols which include between one and four incubations with probes and substrates.

EP 0 276 134 A2

## Description

### MULTICOLOR WESTERN BLOT

### BACKGROUND OF THE INVENTION

This invention relates to methods for detecting proteins, protein fragments, glycoproteins, lipoproteins, or mixtures thereof (hereinafter referred to generally as "proteins"). More particularly, the invention relates to the Western blot and dot blot techniques for protein detection.

As known in the art, a Western blot is formed when proteins are transferred from an electrophoresis gel to a protein adsorbing matrix which immobilizes the transferred proteins at locations corresponding to their locations in the gel. See Gershoni et al., "Protein Blotting: Principles and Application", Analytical Biochemistry, 131, 1-15 (1983), the relevant portions of which are incorporated herein by reference.

Once immobilized on the protein adsorbing matrix, the locations of particular proteins are determined by incubating the matrix with a probe, i.e., a binding partner specific to one or more of the proteins whose presence or location on the matrix is to be determined. For example, the matrix can be incubated with an antibody, an antigen, a lectin, or the like.

The probe is either labelled prior to being incubated with the matrix or can be labelled once on the matrix by reaction with a second labelled probe specific to the first probe, e.g., unlabelled antibodies to a specific protein from a first animal can be labelled on the matrix through the use of a labelled antibody from a second animal, the labelled antibody from the second animal binding generally to antibodies from the first animal. Labels which have been used include radiolabeling, conjugation to an enzyme, and conjugation to a fluorescent tag. See Gershoni et al., supra, at page 9. In the case of radiolabeling, an autoradiograph of the blot is prepared by contacting the blot with a sheet of photographic film thereby exposing the film at the places at which the radioactive probe has become bound to the blot.

Although a number of approaches have been proposed and tested, prior to the present invention, there has not been a convenient, practical method for determining the locations in an electrophoresis gel of a plurality of proteins by means of the Western blot technique. For example, multiple transfers from a gel to a series of matrices followed by development of individual matrices with different probes has been used. See Legocki, et al., "Multiple Immunoreplica Technique: Screening for Specific Proteins with a Series of Different Antibodies Using One Polyacrylamide Gel", Analytical Biochemistry, 111, 385-392 (1981); Lin et al., "Qualitative and Quantative Analyses of Epstein-Barr Virus Early Antigen Diffuse Component by Western Blotting Enzyme-Linked Immunosorbent Assay with a Monoclonal Antibody", Journal of Virology, 53, 793-799 (1985). Plainly, the need to make multiple transfers makes this technique cumbersome. See Erickson et al., "Quantitative Electrophoretic Transfer of Polypeptides from SDS Polyacrylamide Gels to Nitrocellulose Sheets: A Method for Their Re-Use in Immunoautoradiographic Detection of Antigens", Journal of Immunological Methods, 51, 241-249 (1982) at page 248. Moreover, as recognized in the art, it is difficult to ensure that all of the blots will faithfully represent the original protein pattern in the gel. See Gershoni et al., supra, at page 8.

In order to avoid the multiple transfer problem, techniques employing radiolabels have been developed wherein 1) a blot is developed with a first probe, 2) the blot is autoradiographed, and 3) the first probe is eluted from the blot, following which the process is repeated with one or more additional probes. See Erickson et al., supra; Gershoni et al., supra, at pages 9-10; Gullick et al., "Structural Similarities between Acetylcholine Receptors from Fish Electric Organs and Mammalian Muscle", Biochemistry, 21, 4563-4569 (1982); Legocki et al., supra; Olmsted, J. B., "Affinity Purification of Antibodies from Diazotized Paper Blots of Heterogeneous Protein Samples", The Journal of Biological Chemistry, 256, 11955-11957 (1981); Reiser et al., "Immunological Detection of Specific Proteins in Total Cell Extracts by Fractionation in Gels and Transfer to Diazophenylthioether Paper", Eur. J. Biochem., 114, 569-575 (1981); Renart et al., "Transfer of Proteins from Gels to Diazobenzyloxymethyl-paper and Detection with Antisera: A Method for Studying Antibody Specificity and Antigen Structure", Proc. Natl. Acad. Sci. USA, 76, 3116-3120 (1979); Symington et al., "Immunoautoradiographic Detection of Proteins after Electrophoretic Transfer from Gels to Diazo-paper: Analysis of Adenovirus Encoded Proteins", Proc. Natl. Acad. Sci. USA, 78, 177-181 (1981). Although better than the multiple blot approach, this multiple autoradiograph procedure has its own problems, the primary one being the difficulty in aligning a series of autoradiographs to determine the exact location of individual bands. As will be evident, this problem will be most severe when trying to discriminate between proteins having similar molecular weights. Moreover, as with the multiple blot approach, the multiple autoradiograph procedure is fundamentally cumbersone due to the need to prepare, identify, and store multiple records relating to a single gel.

In addition to the foregoing, in one reported study, a blot was probed with a first specific probe and developed using a conjugated enzyme, and then probed with a second general probe (Con A) and again developed using the same conjugated enzyme. See Anderson et al., "Specific Antiserum Staining of Two-dimensional Electrophoretic Patterns of Human Plasma Proteins Immobilized on Nitrocellulose", Electrophoresis, 3, 135-142 (1982). In this study, the investigators generally knew beforehand the locations on the blot of the various proteins they were studying. Accordingly, they were able to interpret their blot notwithstanding the fact that both probes were developed to the same color. Indeed, their purpose in using the second probe was to provide a frame of reference for identifying the location of the first probe. Without such prior knowledge, the locations of the two probes could not have been determined using the Anderson et al. technique.

The development of a single blot using multiple probes has also been discussed in Geysen et al., "How to perform subsequent or 'double' immunostaining of two different antigens on a single nitrocellulose blot within one day with an immunoperoxidase technique," Electrophoresis, 5:129-131 (1984), Lin and Pagano, "Sequential Detection of Different Antigens Induced by Epstein-Barr Virus and Herpes Simplex Virus in the Same Western Blot by Using Dual Antibody Probes," Journal of Virology, 59:522-524 (1986), and Theisen et al., "Sequential Detection of Antigens in Western Blots with Differently Colored Products," Analytical Biochemistry, 152:211-214 (1986).

In each of these references, as in the Anderson reference, the blot is sequentially developed. Specifically, in accordance with each of the Geysen, Lin and Theisen techniques, the blot is sequentially incubated with 1) a first antibody which is specific to one of the antigens to be detected, 2) an enzyme-labeled antibody which binds to the first antibody, 3) a substrate for the enzyme label, 4) an eluting solution to elute the first antibody from the blot, 5) a second antibody which is specific to the other of the antigens to be detected, 6) an enzyme-labeled antibody which binds to the second antibody, and 7) a substrate for the enzyme label. The sequential development makes all of these techniques complicated and slow. Specifically, Geysen et al. state that their technique involves 17 steps and requires a full working day (about 6 hours) to perform; Lin and Pagano describe 12 steps which take over 10 hours to perform; and Theisen et al. describe more than 12 steps which take about 24 hours to perform (2.5 hours for each of the four antibody incubations plus an overnight incubation in BSA).

In addition to the Western blot techniques, immunostaining of proteins immobilized on a protein adsorbing matrix has also been performed as part of the dot blot procedure. In accordance with this procedure, the protein in a series or group of samples is extracted from the samples and localized on specific areas of a sheet of filter paper by placing the samples in a sample well plate which is located over the filter paper and applying vacuum to the bottoms of the wells to draw the samples through the filter paper. See, for example, Schleicher and Schuell's product brochure entitled "MINIFOLD - Mircro-Sample Filtration Manifold," Schleicher and Schuell, Inc., Keene, N.H. (1981). In the past, immunostaining of the filter paper to determine if particular samples contain proteins of interest has been performed using techniques similar to those employed with Western blots. In particular, with regard to the present invention, simultaneous immunostaining of dot blots with multiple probes and enzyme substrates has not been performed.

SUMMARY OF THE INVENTION

In view of the foregoing state of the art, it is an object of the present invention to provide an improved method for using the Western blot procedure to identify the locations on an electrophoresis gel of multiple proteins. More particularly, it is an object of the invention to provide a method for identifying multiple proteins on a single Western blot without the need for multiple blots or multiple autoradiographs. It is a further object of the invention to minimize the number of steps and the time required to perform the identification.

It is also an object of the invention to provide a method for simply and rapidly identifying multiple proteins on dot blots. As with the Western blots, it is an object of the invention to minimize the number of steps and time required to provide the identification.

To achieve the foregoing and other objects, the invention, in accordance with certain of its embodiments, provides a method for developing a Western or dot blot comprising the steps of:

(a) incubating the blot with a mixture of first and second unlabelled probes which bind to different proteins (hereinafter referred to as "binding probes");

(b) incubating the blot with a mixture of first and second labelled probes (hereinafter referred to as "marking probes"), the first marking probe binding specifically to the first binding probe and the second marking probe binding specifically to the second binding probe, the first marking probe being labelled with a first enzyme and the second marking probe being labelled with a second enzyme;

(c) marking the location or locations of the first marking probe on the blot by incubating the blot with a first substrate which, in the presence of the first enzyme, produces a first marker substance having a first color; and

(d) marking the location or locations of the second marking probe on the blot by incubating the blot with a second substrate which, in the presence of the second enzyme, produces a second marker substance having a second color, the first and second colors being different.

As used in the specification as well as in the appended claims, the term "marking probe" is intended to include one-part probes in which the enzyme is carried on the molecular species which binds to the binding probe and two-part probes in which a "bridging probe" is used to connect the binding probe to a "labelled probe" which carries the enzyme. For example, a binding probe/two-part marking probe combination can comprise a monoclonal antibody from a first animal which binds to the protein of interest (binding probe), an unlabelled antibody from a second animal which binds generally to antibodies from the first animal (bridging probe), and a monoclonal antibody from the first animal which binds to the enzyme which is to be used to mark the positions of the protein on the blot (labelled probe). A discussion of probes of this type can be found in Accurate Chemical's product brochure entitled "(PAP) Peroxidase-Anti- Peroxidase Complexes," Accurate Chemical and Scientific Corporation, Westbury, New York.

When two-part probes are used, the blot is incubated with first and second bridging probes which bind to the first and second binding probes, respectively, washed, and then incubated with first and second labelled probes which bind to the first and second bridging probes, respectively, and which carry the first and second enzymes, respectively. Depending on the specific probes being used and the protein being detected, the washing step between the application of the bridging probes and the labelled probes can, in some cases, be omitted. In these cases, the bridging probes are applied to the blot first and then the labelled probes. Alternatively, the bridging and labelled probes can be applied simultaneously.

If desired, two-part marking probes can be used in combination with one-part marking probes, e.g., one protein can be detected with a one-part marking probe and a second protein can be detected with a two-part marking probe. In this case, all of the probes can be applied to the blot together or the one-part probe and the bridging probe of the two-part probe can be applied together and the labelled probe of the two-part probe applied later, with or without intermediate washing of the blot.

In certain embodiments, steps (a) and (b) of the foregoing method are combined so that the method involves only a single incubation with the probes. Similarly, by proper selection of the first and second enzymes and the first and second substrates, marking steps (c) and (d) can be performed simultaneously. In addition, if desired, the probe incubation and marking steps can be performed simultaneously. Accordingly, the invention can be practiced in various formats involving between one and four incubations with probes and substrates.

In addition to the foregoing method, the binding probes can be directly labelled using various enzyme coupling techniques known in the art. In this case, the method of the invention comprises the following steps:

(a) incubating the blot with a mixture of first and second labelled probes which bind to different proteins, (hereinafter referred to as "binding-marking probes"), the first binding-marking probe being labelled with a first enzyme and the second binding-marking probe being labelled with a second enzyme;

(b) marking the location or locations of the first binding-marking probe on the blot by incubating the blot with a first substrate which, in the presence of the first enzyme, produces a first marker substance having a first color; and

(c) marking the location or locations of the second binding-marking probe on the blot by incubating the blot with a second substrate which, in the presence of the second enzyme, produces a second marker substance having a second color, the first and second colors being different.

Again, through the proper selection of the first and second enzymes and the first and second substrates, marking steps (b) and (c) can performed simultaneously to produce a two-step process. Similarly, if desired, the probe incubation and marking steps can be combined to produce a one-step process.

As will be evident, the locations of additional proteins can be determined by developing the blot with additional probe/enzyme/substrate combinations which produce products having colors not previously used to develop the blot. The locations of the additional proteins can be determined without eluting the probes for the original set of proteins from the blot, or the original probes can be eluted and the blot redeveloped for the additional proteins using the techniques of the present invention and/or those of the prior art. It should be noted that if more than one probe binds to the same protein, hybrid colors can be produced.

In addition to its use in localizing different proteins on Western or dot blots, the method of the invention can be used in a variety of protein binding studies employing Western or dot blot techniques.

For example, the technique can be used to compare two or more antibodies with regard to which epitopes the antibodies recognise on a particular antigen. This study is performed by, for example, digesting the antigen into fragments, separating the fragments on an electrophoresis gel, transferring the fragments to a blot, incubating the blot with first and second antibodies, and marking the locations of the first and second antibodies with first and second enzyme/substrate systems which produce first and second marker substances having different colors. Determining whether the two antibodies recognise the same or different epitopes then becomes a matter of simply examining the blot to see if the first and second marker substances appear at the same or different locations.

Similarly, the technique can be used to study the binding of membrane receptor proteins to antigens, hormones, neurotransmitters, and the like. This study is performed by, for example, digesting the membranes into fragments, separating the fragments on an electrophoresis gel, transferring the fragments to a blot, incubating the blot with, for example, first and second antigens, incubating the blot with first and second antibodies to the first and second antigens, respectively, and marking the locations on the blot of the first and second antibodies with first and second enzyme/substrate systems which produce first and second marker substances having different colors. The diferences in binding of the membrane fragments to the two antigens will then be readily apparent from the color distribution of the two marker substances on the blot.

Other applications of the method of the present invention to the study of protein binding will be evident to persons skilled in the art in view of the present disclosure.

In addition to their use in detecting multiple proteins on protein absorbing matrices, the techniques of the invention can also be used to detect a single protein on such matrices. Specifically, as illustrated in Examples 4 and 5 below, the elimination of various of the wash steps used in the prior art and the combination of various of the incubation steps results in extremely rapid and easily-performed methods for detecting specific proteins of interest on protein absorbing matrices. In the past, persons working in the art felt that all of the intermedite wash and incubation steps were essential to a successful development of a protein absorbing matrix. The elimination of these intermediate steps, as demonstrated in the present specification, forms part of the overall scope of the invention.

## DESCRIPTION OF THE PREFERRED EMBODIMENTS

As discussed above, the present invention relates to an improved method for developing Western and dot blots.

The Western and dot blots are produced by conventional techniques known in the art. Thus, Western blots are prepared by electrophoretically separating a mixture of proteins on an electrophoresis gel, such as, a SDS-polyacrylamide gel, and then transferring the separated proteins to a protein adsorbing matrix, such as, a nitrocellulose filter membrane. The transfer can be performed electrophoretically, by diffusion, or by mass flow of solvent, electrophoretic transfer being preferred.

Similarly, dot blots are prepared using commercially available equipment by placing protein-containing samples in the wells of a sample plate and vacuum filtering the samples through a protein adsorbing matrix, e.g., a nitrocellulose filter, so as to transfer the proteins in the sample to the matrix.

The dot blot and Western blots so produced are then developed using the method of the invention. In the case of dot blots, the development can be performed using the dot blot equipment or the filter can be removed from the apparatus and processed separately.

The method of the invention can be practiced in various formats. For example, when separate binding and marking probes are used, the detection of two proteins can involve between one and four incubations. In the four incubation embodiment, the blot is 1) incubated with two binding probes for the two proteins of interest for a period of, for example, 1-2 hours and then washed; 2) incubated with two marking probes for a period of, for example, 1/2 - 1 hour and then washed; 3) incubated with a first substrate for a period of, for example, 5 minutes and then washed; and 4) incubated with a second substrate for a similar period and again washed. Additional proteins can be detected by simply including additional binding and marking probes in the first two incubations and adding an additional substrate incubation for each additional protein which is to be detected. In general, washing of the blot takes about 5 minutes and is performed with, for example, TBS. Accordingly, for the two protein/four incubation embodiment, the overall development process takes between about 2 and about 3.5 hours to perform.

The required time and the number of steps involved can be reduced by combining various of the incubations. For example, the binding probe and marking probe incubations can be combined into a single 1-2 hour incubation followed by a single wash, thus reducing the overall development time to between approximately 1.5 and 2.5 hours. (Note that the marking probes do not have to be present throughout the incubation, but can be added towards the end of the incubation, if desired.) Similarly, the substrate incubations can be combined to further reduce the number of steps and, to some extent, the time involved in the development process. In making this combination, substrates must be selected which are compatible with one another. In particular, the substrates must be soluble in a single buffer, e.g., at a common pH. Examples of compatible enzyme/substrate systems include peroxidase and alkaline phosphatase enzymes with their respective substrates.

Even further simplification and time reduction can be achieved by simultaneously incubating the blot with both the probes and substrates without any intermediate washes. The probes and substrates can be applied to the blot together or the probes can be applied first (with, for example, the binding probes being applied before the marking probes) and the substrates applied thereafter. Due to the presence of unbound marking probe in the incubation solution, substrates will be converted to marker substances both in the solution and on the blot. Accordingly, this embodiment generally requires high amounts of substrate in order to generate levels of marker substance on the blot which can be easily visualized. Conversion in the incubation solution can be minimized by separating the blot from the bulk of the solution prior to the addition of the substrate. By means of this one-step procedure, blots can be developed in times on the order of an hour or less, with a minimum number of manipulations of the blot.

If the binding probes are directly labelled, the detection of two proteins can involve between one and three incubations. In the three incubation embodiment, the blot is 1) incubated with the two binding-marking probes for a period of, for example, 1-2 hours and then washed; 2) incubated with the first substrate for a period of, for example, 5 minutes and then washed; and 3) incubated with the second substrate for a similar period and again washed. Accordingly, for this embodiment, the overall development process takes between about 1.5 and about 2.5 hours to perform. Additional proteins can be detected by simply including additional binding-marking probes in the first incubation and adding an additional substrate incubation for each additional protein which is to be detected.

As with the binding probe/marking probe embodiments, the number of steps and time required can be reduced by combining the substrate incubations with each other, and by combining the probe incubation with the substrate incubations. The considerations discussed above regarding these combinations of incubation steps also apply to the binding-marking probe embodiments.

In connection with each of the foregoing embodiments, it is preferred to incubate the matrix with a protein-containing blocking buffer, e.g., a buffer containing BSA, prior to the beginning of the development process. Such a buffer blocks sites on the matrix which have not become bound to protein during the protein transfer process. For the same reason, a protein such as BSA can be included in the buffers for the probes. An incubation with the blocking buffer for a period of approximately 30 minutes is generally sufficient, although longer incubation periods can be used if desired.

The binding probes or binding-marking probes are selected so as to bind more strongly to the proteins or groups of proteins whose presence and/or location on the matrix is to be determined than to other proteins on the matrix. Depending on the particular study being performed, these probes can comprise antibodies, antigens, lectins, or similar substances capable of binding to proteins. Also, in some cases, mixtures of substances, such as, mixtures of antibodies or antigens, can be used. Following procedures known in the art, the incubation conditions are adjusted to minimize binding or the probes to substances other than the proteins or groups of proteins of interest.

In some cases, it may be convenient to use a series of probes to mark the location of the protein of interest, each probe binding to a previous probe, and the last probe being conjugated to an enzyme. For example, in the case of the membrane receptor study discussed above, the first probe can comprise a hormone to which is bound an unconjugated first antibody to the hormone, to which, in turn, is bound a conjugated second antibody to the first antibody.

For each of the embodiments of the invention, incubation with the substrate results in the formation of a marker substance which binds to or reacts with the matrix, thus forming a permanent identification of the location of the proteins or groups of proteins of interest. Because the substrates are applied to the blot with both marking probes or both binding-marking probes present on the blot, different enzymes must be used for each probe.

A variety of enzyme/substrate systems can be used in the practice of the invention. The enzyme should produce a product which is insoluble in its buffer, which has a clear, distinctive color, and which precipitates directly at the site at which it is produced. The enzyme itself must be active after coupling to the probe, should preferably be stable at room temperature, and should preferably be readily obtainable at a reasonable cost. Horseradish peroxidase and alkaline phosphatase from E. coli or calf intestines have been found to satisfy these requirements. Glucose oxidase from Aspergillus niger, lactate dehydrogenase from pig muscle and heart, and acid phosphatase can also be used, but are less preferred. See Blake et al. (1984) Anal. Biochem., 136, 175-179; Nakane et al. (1966) J. Histochem. Cytochem., 14, 789-790; Pearse 91961) Histochemistry -- Theoretical and Applied, Brown and Co., Boston, MA.

Table 1 sets forth a list of selected colors which can be produced by appropriate selection of the conjugated enzyme and its substrates.

In addition to identifying additional proteins by adding additional probes to the initial incubations, additional proteins can also be identified by eluting the original probes from the matrix using, for example, an acidic buffer or a buffer containing a denaturing agent, such as, urea, SDS, or potassium thiocyanate, and then redeveloping the blot for the additional proteins. By means of this approach, one or more of the enzymes used in the first development of the blot can be reused in the second development with different substrates to generate different marking substances having different colors. Similarly, binding antibody/marking antibody combinations from the same pair of animals, e.g., mouse/goat, can be employed in both the first and second developments. This ability to use the same animal pairs is of particular value when monoclonal antibodies are to be used since the number of animals from which these antibodies can currently be obtained is limited.

Without intending to limit it in any manner, the present invention will be more fully described by the following examples. The materials and methods which are common to the examples are as follows.

Materials and Methods

Strains and Plasmids

E. coli K12 strain JA221 (hsdM+ hsdR- recA leu lacY trp) was used as the transformation host strain. Transformation was performed with plasmid pNL008 which harbours an expression plasmid containing inserts of DNA sequences coding for human alpha-1 interferon.

Antigens, Antibodies and Reagents

Recombinant alpha-1 interferon was extracted from cell cultures of E. coli strains JA221/pNL008 following the procedures described below. Recombinant alpha-2 interferon, natural gamma interferon and polyclonal rabbit antibodies against gamma interferon were obtained from Interferon Sciences Incorporated, New Brunswick, New Jersey (hereinafter "ISI"). Monoclonal rat antibody YOK against alpha-1 interferon and mouse antibody NK2 against alpha-2 interferon were obtained from Celltech Limited, Berkshire, UK. The marking (developing) antibodies, peroxidase-conjugated goat-anti-mouse IgG, peroxidase-conjugated goat-anti-rat IgG, and alkaline phosphatase-conjugated goat-anti-rabbit IgG were from Bio-Rad Laboratories, Richmond, California. The substrates, 4-chloro-1-naphthol, 3,3'-diaminobenzidine and 5-bromo-4-chloro-3-indoxyl phosphate, were from Sigma Chemical Company, St. Louis, Missouri.

Extraction and Purification of Alpha-1 Interferon

The cell extract from a 1,000 ml culture of E. coli JA221/pNL008 at $OD_{600} = 1.0$ was prepared according to the method described in Lee, et al., "Cloning with tandem gene systems for high level gene expression," Nucl. Acid Res., 12:6797 (1984). The cell extract was dialysed overnight against 1 x PBS buffer and loaded onto an alpha interferon affinity chromatographic column, followed by consecutive washing with 1 x PBS, PBS/Ethylene Glycol (30% PBS, 1.5 M NaCl, 50% ethylene glycol) and 1 x PBS. The recombinant alpha-1 interferon was eluted from the column with glycine-HCl buffer (pH 2.2). The eluates were neutralized with solid Tris-base, dialysed against 0.1 M ammonium bicarbonate, lyophilized, and stored at -20°C.

## Electrophoresis and Blotting

SDS-polyacrylamide gel electrophoresis was performed using a 15% slab gel by the method of Anderson et al., (1973), J. Virol, 12, 241-252. After electrophoresis, Western blots were formed by electrophoretically transferring the proteins from the gel to nitrocellulose filters in blotting buffer (15.6 mM tris, 120 mM glycine, pH 8.3, 20% methanol) at 70 V for 3 hours at 4°C. See Towbin et al., (1979), Proc. Natl. Acad. Sci. (U.S.A.), 76, 4350-4354. The filters used were BA85 nitrocellulose filters (0.45 micron) manufactured by Schleicher and Schuell, Keene, New Hampshire.

## Preparation of substrate solutions

100 ml of each of the substrate solutions were freshly prepared as follows: a) 12.5 mg of diaminobenzidine (DB) was dissolved in 100 ml of TBS, and 100 ul of 30% $H_2O_2$ was added just before use; b) 50 mg of chloronaphthol (CP) was dissolved in 10 ml of absolute ethanol or methanol and 90 ml of TBS, and 0.25 ml of 30% $H_2O_2$ were mixed in just before use; and c) 9.2 mg of bromochloroindoxylphosphate (BCIP) was dissolved in 35 ml of 0.25 M glycine-NaOH buffer, pH 10.4, and then mixed with 1.25 ml of 0.1 M $ZnCl_2$, 1.25 ml of 0.1 M $MgCl_2$ and 62.5 ml of distilled water (the glycine-NaOH buffer mixed with the $ZnCl_2/MgCl_2$ solution is hereinafter referred to as "buffer A").

## Example 1

This example describes an exemplary set of conditions for marking a Western blot with two marker substances having different colors. The four-incubation protocol described above, e.g., a binding probe incubation, a marking probe incubation, a 1st substrate incubation, and a 2nd substrate incubation, is used.

1.0 ug each of recombinant alpha-1 interferon and natural gamma interferon are subjected to electrophoresis on SDS-PAGE, followed by blotting onto a nitrocellulose membrane. The blotted nitrocellulose membrane is washed with TBS (20 mM Tris-HCl, pH 7.5; 500 mM NaCl) and blocked with T-TBS (TBS + 0.05% Tween 20) containing 3% BSA for 30 min at room temperature.

The membrane is immunostained with a mixture of YOK rat monoclonal antibody against alpha-1 interferon (1:10,000) and rabbit polyclonal antibody against human gamma interferon (1:100) for 1-2 hours. The membrane is washed in T-TBS for 5 minutes and then incubated for 1/2-1 hours in a mixture of peroxidase-conjugated goat-anti-rat IgG (1:1000) and alkaline phosphatase-conjugated goat-anti-rabbit IgG (1:1000). The membrane is washed again for 5 minutes in T-TBS and then sequentially treated (with intermediate washes) with the DB and BCIP solutions described above, and then given a final wash with TBS or distilled water.

The resulting developed blot containss one brownish green and three turquoise bands, representing alpha-1 interferon and gamma interferon, respectively. The three gamma interferon bands represent different levels of glycosylation of the interferon protein.

## Example 2

This example illustrates the use of a three-incubation protocol (binding probe incubation, marking probe incubation, and combined 1st and 2nd substrate incubation) to develop a dot blot.

$6 \times 10^4$ units of gamma interferon and alpha-1 interferon were added to wells 1 and 2, respectively, of a Schleicher and Schuell MINIFOLD micro-sample filtration manifold and vacuum transferred to a sheet of nitrocellulose filter paper. Following the procedures described in Example 1, the blotted filter was sequentialy incubated with 1) a mixture of YOK rat monoclonal antibody against alpha-1 interferon (1:10,000) and rabbit polyclonal antibody against human gamma interferon (1:100), 2) a mixture of peroxidase-conjugated goat-anti-rat IgG (1:1000) and alkaline phosphatase-conjugated goat-anti-rabbit IgG (1:1000), and 3) a mixture of 25 mg CP dissolved in 5 ml of 100% ethyl alcohol and 5 mg BCIP dissolved in 45 ml of buffer A. The blot was washed for 5 minutes in T-TBS between incubations.

The developed blot had blue color and purple color developed in the well 1 and well 2 positions, respectively.

## Example 3

This example illustrates a three color development of a Western blot, the first two colors being developed without intervening elution of binding and marking probes, and the third color being developed after elution of the binding and marking probes used in generating the first two colors.

1.0 ug each of gamma interferon, recombinant alpha-1 interferon, and recombinant alpha-2 interferon were electrophoresed and then transferred onto a nitrocellulose membrane. Following the procedures of Example 1, the membrane was sequentially incubated with 1) a mixture of rabbit polyclonal antibody against gamma interferon (1:100) and mouse monoclonal antibody NK2 against alpha-2 interferon (1:10,000), 2) a mixture of peroxidase-conjugated goat-anti-mouse IgG (1:1000) and alkaline phosphatase-conjugated goat-anti-rabbit IgG (1:1000), 3) the diaminobenzidine solution described above, and 4) the bromochloroindoxylphosphate solution described above. The membrane was washed with T-TBS for 5 minutes after each incubation. After these incubations and washes, the blot had one brown and three turquoise bands, representing alpha-2 and gamma interferon, respectively.

To remove the original binding and marking probes, the membrane was treated three times with an elution buffer (0.1 M Glycine-HCl, pH 2.2; 20 mM $MgAc_2$; 50 mM KCl). Each was was for 5 minutes. After elution, the

7

membrane was sequentially incubated (with intermediate washes) with YOK monoclonal antibody against alpha-1 interferon (1:10,000), peroxidase-conjugated goat-anti-rat IgG (1:1000), and the chloronaphthol solution described above. Purple color appeared at the blot region containing alpha-1 interferon.

The final three color blot included brown, turquoise, and purple bands representing alpha-2, gamma, and alpha-1 interferon, respectively.

Example 4

This example illustrates the development of dot blots without intermediate washing of the blot between the probe and substrate incubations.

Three dot blots were prepared by transferring $6 \times 10^4$ units of alpha-2 interferon to each of three sheets of nitrocellulose filter paper using the Schleicher and Schuell dot blot apparatus of Example 2. Each blot was blocked with 3% BSA blocking buffer for 15 minutes and washed with T-TBS for five minutes. The blots were then incubated with a mixture of NK2 mouse monoclonal antibody against alpha-2 interferon (1:2500) and peroxidase-conjugated goat-anti-mouse IgG (1:1000) for 1-2 hours.

For the first blot, the diaminobenzidine (DAB) solution described above was added directly to the probe incubation solution, the blot was incubated in this mixture for five minutes and then washed with T-TBS; for the second blot, the blot was removed from the probe incubation solution, placed in the DAB solution without prior washing, incubated in the DAB solution for 5 minutes and then washed with T-TBS; for the third blot, the blot was washed with T-TBS for five minutes, placed in the DAB solution for five minutes and then washed with T-TBS.

Brown color appeared at the location of the alpha-2 interferon for each of the three blots. The color levels for the second and third blots were equivalent; the color level for the first blot was poorer than that for the other blots and the first blot showed some background staining.

This example demonstrates that the location of proteins on filter membranes can be rapidly determined with a minimum number of incubations and washes. The overall time for the development, including incubation with the blocking buffer, is on the order of 1.5 to 2.5 hours. For comparison, if the blots of this example had been developed with the existing techniques for developing filter membranes, e.g., the Geysen et al. technique, supra, the overall development time would have been greater than 2.5 hours (assuming a 0.5 hour incubation with the blocking buffer, a 1 hour incubation with the binding probe, a 0.5 hour incubation with the marking probe, and 5 minutes of washing between the incubations) and the overall process would have involved significantly more manipulations of the blot.

Example 5

This example illustrates a high speed, minimum step technique for developing a dot blot or other protein absorbing matrix.

A dot blot was prepared by transferring $6 \times 10^4$ units of alpha-2 interferon to a sheet of nitrocellulose filter paper using the Schleicher and Schuell dot blot apparatus of Example 2. The blot was blocked with 3% BSA blocking buffer for 5 minutes and washed with T-TBS for five minutes. The blot was then incubated with a mixture of NK2 mouse monoclonal antibody against alpha-2 interferon (1:1000) and peroxidase-conjugated goat-anti-mouse IgG (1:100) for 9 minutes. The higher antibody concentrations allowed the incubation times to be reduced without significant reduction in the intensity of developed color. If desired, higher incubation temperatures and/or a combination of higher antibody concentrations and higher incubation temperatures can be used to shorten the incubation times.

After incubation with the antibodies, the blot was removed from the incubation solution and placed into the DAB solution described above for 1 minute. Brown color appeared on the blot at the location of the alpha-2 interferon.

As illustrated by this example, by eliminating wash steps and combining incubation steps, dot blots and other protein absorbing matrices can be developed in times on the order of 20 minutes or less. Such rapid immunostaining is of particular value in diagnostics and other applications that require rapid detection and measurement. For example, using the foregoing techniques and a protein absorbing matrix in the form of a dip stick, the presence of a particular protein in a solution can be rapidly and simply determined by applying a sample of the solution to the dip stick and then developing the dip stick in the manner described above. Quantification of the amount of the particular protein in the solution can be accomplished by, for example, measuring the color intensity of the developed dip stick using a colorimeter.

## TABLE 1

| ENZYME | PRIMARY SUBSTRATE | COLOR |
|---|---|---|
| Peroxidase | Diaminobenzidine | brown |
| Peroxidase | Aminothylcarbazol | red |
| Peroxidase | Chloronaphthol | purple |
| Peroxidase | Diaminobenzidine/$CoCl_2$ | black |
| Alkaline Phosphatase | 5-bromo-4-chloroindoxyl phosphate (and nitro blue tetrazolium) | blue turquoise |
| beta-galactosidase | X-gal | turquoise blue |
| Peroxidase and beta-galactosidase | Diaminobenzidine and X-gal | green |

**Claims**

1. A method for developing a protein adsorbing matrix to which has been transferred a plurality of proteins, protein fragments, glycoproteins, lipoproteins or mixtures thereof, comprising the steps of:
    (a) incubating the matrix with a mixture of a first binding probe, which is unlabelled and which binds to one or more of the proteins, protein fragments, glycoproteins, or lipoproteins, and a second binding probe, which is unlabelled and which binds to a different one or more of the proteins, protein fragments, glycoproteins, or lipoproteins proteins;
    (b) incubating the matrix with a mixture of first and second marking probes, the first marking probe binding specifically to the first binding probe and the second marking probe binding specifically to the second binding probe, the first marking probe being labelled with a first enzyme and the second marking probe being labelled with a second enzyme;
    (c) marking the location or locations of the first marking probe on the matrix by incubating the matrix with a first substrate which, in the presence of the first enzyme, produces a first marker substance having a first color; and
    (d) marking the location or locations of the second marking probe on the matrix by incubating the matrix with a second substrate which, in the presence of the second enzyme, produces a second marker substance having a second color, the first and second colors being different.
2. The method of Claim 1 wherein the matrix is washed after step (a) but prior to step (b).
3. The method of Claim 1 or 2 wherein the matrix is washed after step (b) but prior to step (c).
4. The method of Claim 1,2 or 3, wherein steps (a) and (b) are performed simultaneously.
5. The method of Claim 1,2 or 3, wherein the matrix is washed after step (c) but prior to step (d).

6. The method of Claim 5, wherein steps (c) and (d) are performed simultaneously.

7. The method of Claim 1, wherein steps (a) and (b) are performed simultaneously.

8. The method of Claim 1,2,3 or 7 wherein steps (c) and (d) are performed simultaneously.

9. The method of any preceding claim wherein steps (a),(b),(c) and (d) are performed simultaneously.

10. The method of any preceding claim including the additional steps after step (d) of eluting the binding and marking probes from the matrix and incubating the matrix with at least one additional binding probe, marking probe, and substrate.

11. A method for developing a protein adsorbing matrix to which has been transferred a plurality of proteins, protein fragments, glycoproteins, lipoproteins or mixtures thereof, comprising the steps of:

(a) incubating the matrix with a mixture of a first probe, which is labelled with a first enzyme and which binds to one or more of the proteins, protein fragments, glycoproteins, or lipoproteins, and a second probe, which is labelled with second enzyme and which binds to a different one or more of the proteins, protein fragments, glycoproteins, or lipoproteins proteins;

(b) marking the location or locations of the first probe on the matrix by incubating the matrix with a first substrate which, in the presence of the first enzyme, produces a first marker substance having a first color; and

(c) marking the location or locations of the second probe on the matrix by incubating the matrix with a second substrate which, in the presence of the second enzyme, produces a second marker substance having a second color, the first and second colors being different.

12. The method of Claim 11 wherein the matrix is washed after step (a) but prior to step (b).

13. The method of Claim 11 or 12 wherein steps (b) and (c) are performed simultaneously.

14. The method of Claim 11 in which the matrix is washed after step (b) but before step (c).

15. The method of Claim 11 or 12 wherein steps (a),(b) and (c) are performed simultaneously.

16. The method of Claim 11 including the additional steps after step (c) of eluting the first and second probes from the matrix and incubating the matrix with at least one additional probe and substrate.

17. A method for developing a protein adsorbing matrix to which a protein-containing solution has been transferred, comprising the steps of:

(a) incubating the matrix with a solution containing a binding probe which is unlabelled and which binds to one or more constituents of the protein-containing solution;

(b) incubating the matrix with a solution which contains a marking probe which binds specifically to the binding probe and which is labelled with an enzyme; and

(c) without washing the matrix to remove unbound marking probe, marking the location or locations of the marking probe on the matrix by incubating the matrix with a substrate which, in the presence of the enzyme, produces a colored marker substance.

18. The method of Claim 17 wherein the matrix is washed after step (a) but before step (b).

19. The method of Claim 17 wherein steps (a) and (b) are performed simultaneously.

20. The method of Claim 17 wherein prior to step (c), the matrix is removed from the solution containing the marker probe.

21. The method of Claim 19 wherein prior to step (c), the matrix is removed from the solution containing the marker probe.

22. A method for developing a protein adsorbing matrix to which a protein-containing solution has been transferred, comprising the steps of:

(a) incubating the matrix with a solution containing a probe which is labelled with an enzyme and which binds to one or more constituents of the protein-containing solution; and

(b) without washing the matrix to remove unbound probe, marking the location or locations of the probe on the matrix by incubating the matrix with a substance which, in the presence of the enzyme, produces a colored marker substance.

23. The method of Claim 22 wherein prior to step (b), the matrix is removed from the solution containing the probe.